# EUROPEAN PATENT SPECIFICATION

(11) **EP 3 360 574 B1**
(45) Date of publication and mention of the grant of the patent: **01.09.2021**
(21) Application number: 16855498.8
(22) Date of filing: 13.10.2016
(51) Int. Cl.: A61K 31/198, A61K 31/355, A61K 45/00, G01N 33/15, G01N 33/50, A61P 19/00, A61P 29/00, A61P 39/06, A61B 5/055, A61B 5/00

(54) **MEDICINAL COMPOSITION FOR AMELIORATING AND TREATING INTERVERTEBRAL DISC DEGENERATION AND LOW BACK PAIN, AND METHOD FOR SCREENING THERAPEUTIC AGENT**
MEDIZINISCHE ZUSAMMENSETZUNG ZUR VERBESSERUNG UND BEHANDLUNG VON BANDSCHEIBENDEGENERATION UND SCHMERZEN IM UNTEREN RÜCKENBEREICH SOWIE VERFAHREN ZUM SCREENING EINES THERAPEUTISCHEN MITTELS
COMPOSITION MÉDICINALE POUR AMÉLIORER ET TRAITER UNE DÉGÉNÉRESCENCE DE DISQUE INTERVERTÉBRAL ET UNE LOMBALGIE, ET PROCÉDÉ DE CRIBLAGE D'AGENT THÉRAPEUTIQUE

(30) Priority: 13.10.2015 JP 2015201829
(43) Date of publication of application: 15.08.2018
(73) Proprietor: Keio University, Tokyo 108-8345 (JP)
(72) Inventor: MATSUMOTO Morio, Tokyo 160-8582 (JP); NAKAMURA Masaya, Tokyo 160-8582 (JP); FUJITA Nobuyuki, Tokyo 160-8582 (JP); SUZUKI Satoshi, Tokyo 160-8582 (JP); NAKASHIMA Daisuke, Tokyo 160-8582 (JP)
(74) Representative: Prüfer & Partner mbB Patentanwälte · Rechtsanwälte
(86) International application number: PCT/JP2016/080452
(87) International publication number: WO 2017/065245

(56) References cited:
- WO-A1-2015/059623
- WO-A2-2006/031376
- DE-A1-102011 006 425
- JP-A- H07 233 074
- JP-A- 2003 088 329
- JP-A- 2005 509 004
- JP-A- 2008 094 793
- US-A- 4 039 682
- DATABASE MEDLINE [Online] US NATIONAL LIBRARY OF MEDICINE (NLM), BETHESDA, MD, US; 1 June 2008 (2008-06-01), BALTZER WENDY I ET AL: "Randomized, blinded, placebo-controlled clinical trial of N-acetylcysteine in dogs with spinal cord trauma from acute intervertebral disc disease.", XP002791481, Database accession no. NLM18520934
- SUZUKI, S et al.: "Reactive oxygen species are therapeutic targets for intervertebral disc degeneration", 2015 ORS Annual Meeting, March 2015 (2015-03), XP009509598,
- SATOSHI SUZUKI et al.: "Excessive reactive oxygen species are therapeutic targets for intervertebral disc degeneration", The 28th Japanese Society of Cartilage Metabolism Program.Shorokushu, vol. 17, no. 316, February 2015 (2015-02), pages 1-17, XP055380855,
- SATOSHI SUZUKI et al.: "Kassei Sansoshu wa Tsuikanban Hensei ni Taisuru Chiryo Hyoteki to Nariuru ka", The Journal of the Japanese Orthopaedic Association, vol. 89, no. 8, August 2015 (2015-08), page S1781, XP009509605,
- DIMOZI A et al.: "Oxidative stress inhibits the proliferation, induces premature senescence and promotes a catabolic phenotype in human nucleus pulposus intervertebral disc cells", Eur Cell Mater, vol. 30, September 2015 (2015-09), pages 89-103, XP055380858,
- CAI, X Y et al.: "Ropivacaine- and bupivacaine- induced death of rabbit annulus fibrosus cells in vitro: involvement of the mitochondrial apoptotic pathway", Osteoarthritis and Cartilage, vol. 23, no. 10, May 2015 (2015-05), pages 1763-1775, XP055380861,
- TURGUT M et al.: "The effect of exogenous melatonin administration on trabecular width, ligament thickness and TGF-beta1 expressionin degenerated intervertebral disk tissue in the rat", J Clin Neurosci, vol. 13, no. 3, 2006, pages 357-363, XP005396952,
- YANG X et al.: "Antioxidative nanofullerol prevents intervertebral disk degeneration", Int J Nanomedicine, vol. 15, no. 9, 2014, pages 2419-2430, XP055380874,
- YANG D et al.: "Glutathione protects human nucleus pilposus cells apoptosis and inhibition of matrix synthesis", Connective Tissue Research, vol. 55, no. 2, 2014, pages 132-139, XP055380881,
- LI Y et al.: "Piperine mediates LPS induced inflammatory and catabolic effects in rat intervertebral disc", Int J Clin Exp Pathol, vol. 8, no. 6, May 2015 (2015-05), pages 6203-6213, XP055380913,
- XIE, R H et al.: "Mechanism of chlorogenic acid on apoptosis of rat nucleus pulposus cells induced by oxidative stress", Journal of Chinese Medicinal Materials, vol. 37, no. 3, 2014, pages 465-469, XP009509603, & DATABASE MEDLINE retrieved from STN Database accession no. 2015068931
- LIU, Q et al.: "A novel treatment of neuroinflammation against low back pain by soluble fullerol nanoparticles", Spine, vol. 38, no. 17, 2013, pages 1443-1451, XP055380995,
- KEN'ICHIRO KAKUTANI et al.: "Tsuikanban Hensei no Kijo to Saisei no Kanosei -Kiso Kenkyu kara Rinsho Kenkyu made", Journal of Joint Surjery, vol. 34, April 2015 (2015-04), pages 27-39, XP009509608,
- KATSURA, M et al.: "Nom-Gaussian diffusion- weighted imaging for assessing diurnal changes in intervertebral disc microstructure", Journal of Magnetic Resonance Imaging, vol. 40, no. 5, 2014, pages 1208-1214, XP055381017,
- SUZUKI S et al.: "Excessive reactive oxygen species are therapeutic targets for intervertebral disc degeneration", Arthritis Research & Therapy, vol. 17, no. 316, November 2015 (2015-11), XP055381040,
- KIMIHIKO MAKIYAMA: "Inhibitory effect of vitamin E on NO production in nucleus pulposus- derived inflammatory reaction", Kawasaki Medical Journal, vol. 41, no. 1, November 2015 (2015-11), pages 1-7, XP055381044,
- KRUPKOVA, O et al.: "The natural polyphenol epigallocatechin gallate protects intervertebral disc cells fron oxidative stress", Oxidative Medicine and Cellular Longevity, vol. 2016, March 2016 (2016-03), pages 1-17, XP055381045,

## Description

### Technical Field

The present invention relates to a composition for improving intervertebral disc degeneration and diseases, such as low back pain, triggered thereby. Particularly, the present invention relates to a pharmaceutical composition comprising N-acetylcysteine (NAC; hereinafter also referred to as NAC) as an active ingredient. The present invention also relates to a method for quantitatively analyzing intervertebral disc degeneration, etc. using MRI images. The present invention further relates to a method for screening for a therapeutic drug for improving intervertebral disc degeneration and diseases triggered thereby.

The present application is a related application of Japanese Patent Application No. 2015-201829 filed on October 13, 2015 and claims the priority based on this Japanese application. Also, the contents described in the paper of the present inventors, Suzuki, S., et al., Arthritis Res. Ther., 2015, 17: 316, DOI10.1186/s13075-015-0834-8.

### Background Art

Intervertebral discs, which lie between vertebrae, are composed of: the nucleus pulposus located at the middle part; and the annulus fibrosus located at the external part so as to surround the nucleus pulposus. The nucleus pulposus consists of a gel-like substance rich in water, but decreases the water content and loses its elasticity due to aging or the like, causing degeneration. This degenerated state of the intervertebral discs is called intervertebral disc degeneration. If such an aging phenomenon deletes the supporting properties of the intervertebral discs and reduces their cushioning functions, the adjacent nerves may be compressed or burdens may be placed on the adjacent tissues of ligaments, joints, muscles, and the like, causing pain.

According to the study of the research group of the Ministry of Health, Labour and Welfare ("A study of large cohort having regional representativeness of knee pain, back pain and fractures in elderly care and prevention", 2012), estimates are that approximately 28,000,000 people have low back pain in Japan. As low back pain occurs due to diverse causes, intervertebral disc degeneration is considered to trigger low back pain and is reportedly a large factor. Also, intervertebral disc degeneration is known to be associated with disc herniation, spinal canal stenosis, and spinal deformities, in addition to low back pain (Non Patent Literature 1).

Since intervertebral disc degeneration is a phenomenon caused by so-called aging, a typical method for treating manifested symptoms such as low back pain is surgical treatment or symptomatic treatment at present. A basic treatment method of the symptomatic treatment is aimed at alleviating symptoms by medication with antipain drugs or physical therapy using corsets or the like. In short, there is no radical method for treating low back pain or intervertebral disc degeneration itself, which is responsible for low back pain.

Among the antipain drugs currently used, nonsteroidal anti-inflammatory drugs often exhibit adverse reactions such as gastrointestinal disorder and renal damage. Also, analgesics frequently used in recent years also often exhibit adverse reactions such as nausea, constipation, vertigo, and sleepiness. Furthermore, decreasing the number of patients who undergo surgical treatment is also very meaningful from the viewpoint of medical economy. Thus, there is a demand for a drug capable of treating intervertebral disc degeneration.

The present inventor has completed the present invention by revealing that oxidative stress is involved in the pathogenesis of intervertebral disc degeneration. Excessive reactive oxygen species (ROS) has previously been reported to give DNA or proteins oxidative stress and be responsible for various diseases (Non Patent Literature 2). However, no previous report mentions the relation of oxidative stress to intervertebral disc degeneration. The group of the present inventors have found oxidative stress is associated with intervertebral disc degeneration (Non Patent Literatures 3 to 5). The present inventors have further found by a model animal experiment that intervertebral disc degeneration is prevented when procedures for causing intervertebral disc degeneration are carried out after 1-week oral administration of NAC (Non Patent Literature 5).

NAC is an approved therapeutic drug for poisoning caused by the overdose of acetaminophen, which is contained as an active ingredient in approximately 90% of antipyretic analgesics or cold remedies. NAC is effective for preventing severe hepatic damage such as fulminant hepatitis caused by acetaminophen overdose, renal damage, and the like. NAC is a glutathione precursor and increases glutathione storage in the liver. Meanwhile, a main metabolite of acetaminophen is N-acetyl-p-benzoquinone imine having cytotoxicity and is detoxicated by the glutathione storage in the liver. NAC reportedly acts to prevent the depletion of glutathione caused by acetaminophen overdose and detoxicate the metabolism of acetaminophen.

NAC is metabolized into the antioxidant glutathione as mentioned above and is therefore considered to prevent aging ascribable to reactive oxygen species in the body. Thus, NAC is also used as an antiaging supplement.

NAC is administered in advance to model animals prior to procedures for causing intervertebral disc degeneration probably because the production of glutathione suppresses oxidative effects. Thus, the effect of NAC on already developed intervertebral disc degeneration itself is unpredictable. The present inventor has revealed that, unexpectedly, the antioxidant NAC has not only a prophylactic effect but a therapeutic effect on intervertebral disc degeneration.

In general, drugs effective by prophylactic administration do not always have a therapeutic effect, though depending on the mechanisms of action of the drugs. Specifically, according to the studies of the present inventors, NAC can be expected to be effective for preventing the manifestation of symptoms of intervertebral disc degeneration, which is responsible for low back pain and the like, whereas it was uncertain whether NAC would be effective for treating the already manifested symptoms of intervertebral disc degeneration.

The present inventors have completed the present invention by finding that NAC is effective for intervertebral disc degeneration by the administration of NAC after a given period from procedures for causing intervertebral disc degeneration. As mentioned above, the treatment of low back pain probably caused by intervertebral disc degeneration is symptomatic treatment with antipain drugs, and there has been no therapeutic drug for improving intervertebral disc degeneration itself. NAC can serve as a therapeutic drug capable of improving intervertebral disc degeneration itself or preventing aggravation thereof and is therefore an unprecedented revolutionary therapeutic drug. The present inventors have further conducted studies on various antioxidants and consequently revealed that tocopherol (vitamin E) can be expected to be effective for treating intervertebral disc degeneration.

The present inventors have also revealed that oxidative stress is responsible for intervertebral disc degeneration. Hence, a screening method for selecting a therapeutic drug by targeting reactive oxygen species generating oxidative stress can be provided. The revealed cause of the disease has expanded the possibility of obtaining a novel therapeutic drug.

The present inventors have further developed a technique of quantifying and visualizing the degree of intervertebral disc degeneration from MRI images. This technique can not only be used in the examination of intervertebral disc degeneration, but can confirm the effect of the therapeutic drug of the present invention by comparing quantification values before and after treatment. This method can further quantitatively confirm the effect of a candidate substance and is therefore very useful in screening for intervertebral disc degeneration and low back pain improving drugs using model animals.

### Citation List

### Non Patent Literature

Non Patent Literature 1: Urban, J.P., Roberts S. Arthritis Res. Ther. 2003, Vol. 5, p. 120-130
Non Patent Literature 2: Pajares, M., et al., Redox Biology, 2015, Vol. 6, p. 409-420
Non Patent Literature 3: Satoshi Suzuki et al., The 28th Annual Meeting of the Japanese Society of Cartilage Metabolism, February 2015
Non Patent Literature 4: Satoshi Suzuki et al., The 44th Annual Meeting of the Japanese Society for Spine Surgery and Related Research, March 25, 2015
Non Patent Literature 5: Suzuki, S., et al., 2015 ORS Annual Meeting, March 23, 2015
Non Patent Literature 6: Oikawa, D., et al., 2012, Sci. Rep., 2012, Vol. 2, p. 229
Non Patent Literature 7: Callaghan, P.T., et al., Nature, 1991, Vol. 351, pp. 467-469
Non Patent Literature 8: Yamada, I., et al., Magn. Reson. Med., 2015, Vol. 73, pp. 2262-2273
Non Patent Literature 9: Fujiyoshi, K., et al., J. Neurosci., 2016, Vol. 36 (9), pp. 2796-2808
Non Patent Literature 10: Assaf, Y., et al., Magn. Reson. Med., 2000, Vol. 44, pp. 713-722
Non Patent Literature 11: Katsura, M., et al., J. Magn. Reson. Imaging, 2014, Vol. 40, pp. 1208-1214
Non Patent Literature 12: Katsura, M., et al., Proc. Intl. Soc. Mag. Reson. Med. 24, 2016, http://www.ismrm.org/16/program_files/EP20.htm

### Summary of Invention

### Technical Problem

An object of the present invention is to provide a drug for treating intervertebral disc degeneration responsible for low back pain, disc herniation, and spinal canal stenosis. Another object of the present invention is to provide a method for screening for a novel drug, and a method for visualizing and examining intervertebral disc degeneration.

### Solution to Problem

The present invention relates to a composition for treating intervertebral disc degeneration as well as low back pain, disc herniation, and spinal canal stenosis associated therewith by suppressing oxidative stress which is a factor responsible for the pathogenesis of intervertebral disc degeneration. The present invention also relates to a method for screening for a pharmaceutical drug targeting oxidative stress from a newly found mechanism underlying the pathogenesis of intervertebral disc degeneration.
(1) A therapeutic drug for use in a method of treating intervertebral disc degeneration and/or a drug for use in a method of improving low back pain by oral administration comprising an antioxidant as the only active ingredient, wherein the antioxidant is N-acetylcysteine, and is administered at a dose of 10 mg/kg/day to 159 mg/kg/day.
(2) A method for examining a therapeutic effect of the therapeutic drug for intervertebral disc degeneration and/or the low back pain improving drug according to (1), comprising analyzing MRI images obtained before and after treatment by Q-space imaging and comparatively analyzing the quantified values, as defined in claim.
(3) A method for screening for a therapeutic drug for intervertebral disc degeneration, and/or a low back pain improving drug, comprising: contacting a candidate substance with annulus fibrosus (AF) cells under induced oxidative stress; and using a signal induced by reactive oxygen species as an index, and further allowing a rodent intervertebral disc degeneration model animal that has developed intervertebral disc degeneration, to take a candidate substance selected by the screening method and making a selection by using a signal induced by reactive oxygen species as an index.
(4) The method for screening for a therapeutic drug for intervertebral disc degeneration and/or a low back pain improving drug according to (3), further comprising:
   obtaining MRI images of the rodent intervertebral disc degeneration model animal before and after the administration of the candidate substance, analyzing the obtained MRI images by Q-space imaging; and comparatively analyzing the quantified values to determine the efficacy of the candidate substance. According to the invention, the method of examining a disease, which is quantifying the degree of degeneration is a method that acquired MRI images which are analyzed by Q-space imaging using Probability at zero displacement and/or Fractional Anisotropy of Kurtosis.
The disease is intervertebral disc degeneration.

The present disclosure also relates to the following non-claimed aspects. A method of treatment characterized by administering the drug according to (1), until the therapeutic effect can be confirmed by quantifying and comparing the disease by the above examination method before and after the administration. A method of prophylaxis, wherein predicting the onset of a disorder caused by intervertebral disc degeneration using the above examination method, and administering the drug according to (1).

### Advantageous Effects of Invention

As shown in detail below, NAC and tocopherol (tocopherol does not form part of the present invention) are effective for the treatment of intervertebral disc degeneration and are therefore considered effective for the treatment of various diseases, including low back pain, disc herniation, and spinal canal stenosis, which are thought to be triggered by intervertebral disc degeneration. Furthermore, both NAC and tocopherol are drugs with few adverse reactions and can therefore be used safely over a long period.

Moreover, since the present inventors have revealed that oxidative stress is responsible for intervertebral disc degeneration, a novel therapeutic drug for intervertebral disc degeneration can be screened for by using reactive oxygen species as a therapeutic target.

### Brief Description of Drawings

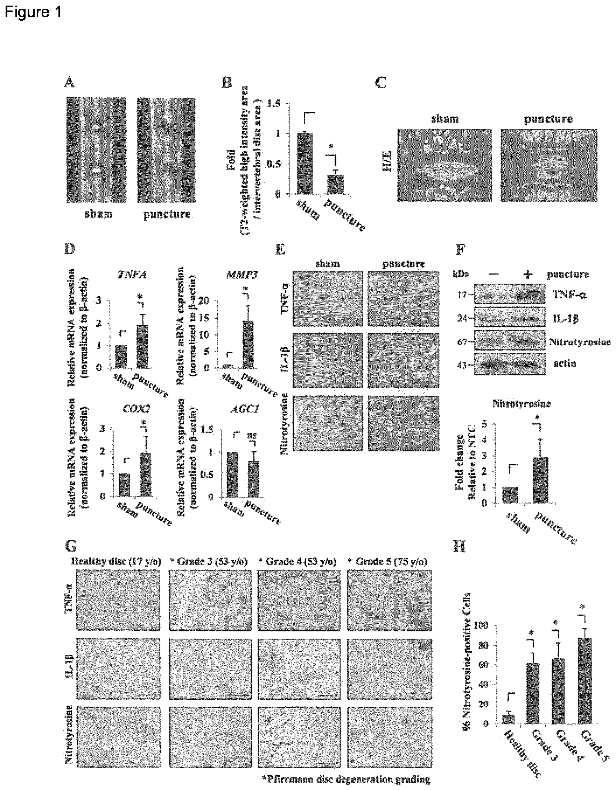

[Figure 1] Figure 1 is a diagram showing change in gene expression, etc. in intervertebral disc degeneration. Figure 1A shows MRI images of rat intervertebral disc degeneration models. Figure 1B shows the ratio of a high-intensity area to intervertebral disc areas in MRI. Figure 1C shows HE staining images of rat intervertebral disc degeneration models. Figure 1D shows differences of gene expressions in intervertebral disc degeneration models. Figure 1E shows histological staining images using oxidative stress markers. Figure 1F shows the analysis of oxidative stress markers by Western blotting. Figure 1G shows histological staining images of human intervertebral disc degeneration patients using oxidative stress markers. Figure 1H shows the quantification of tyrosine nitration in human intervertebral disc degeneration patients.

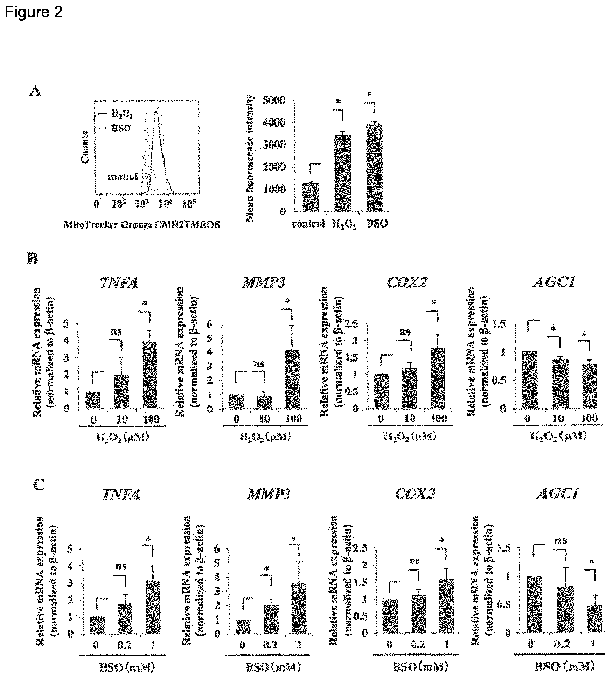

[Figure 2] Figure 2 is a diagram showing results of analyzing the response of rat annulus fibrosus cells to oxidative stress. Figure 2A shows an increase in the intracellular level of reactive oxygen species by oxidative stress. Figure 2B shows change in gene expression by hydrogen peroxide. Figure 2C shows change in gene expression by BSO.

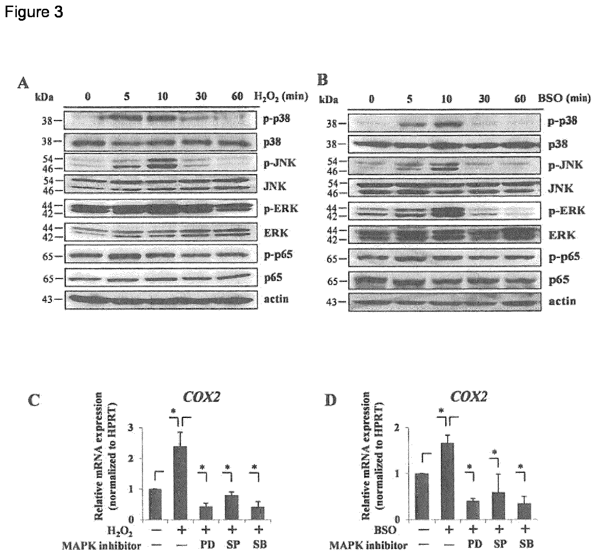

[Figure 3] Figure 3 is a diagram showing results of analyzing downstream signals after application of oxidative stress. Figure 3A shows the analysis of MAPK phosphorylation after addition of hydrogen peroxide. Figure 3B shows the analysis of MAPK phosphorylation after addition of BSO. Figures 3C and 3D show the effects of MAPK inhibitors.

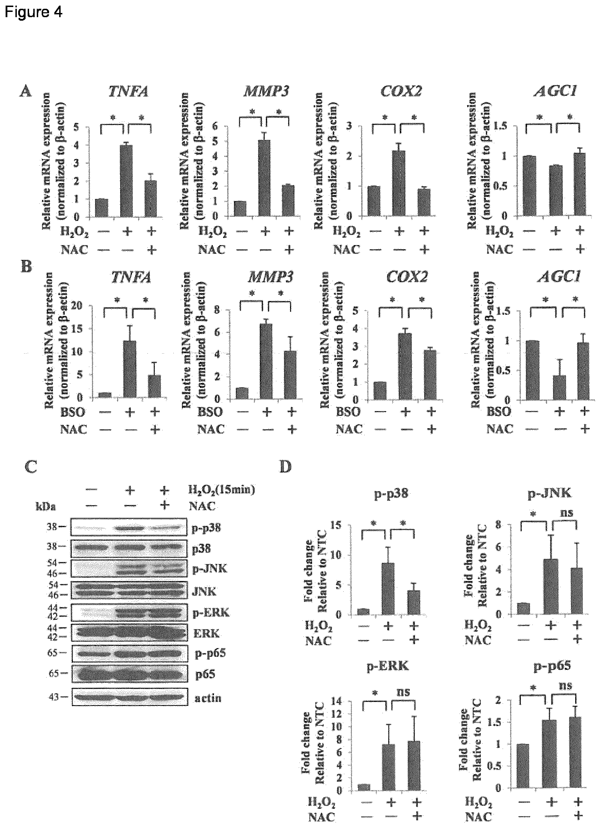

[Figure 4] Figure 4 is a diagram showing the effect of NAC on oxidative stress. Figure 4A shows that NAC reduces oxidative stress induced by hydrogen peroxide. Figure 4B shows that NAC reduces oxidative stress induced by BSO. Figures 4C and 4D show the effect of NAC on MAPK.

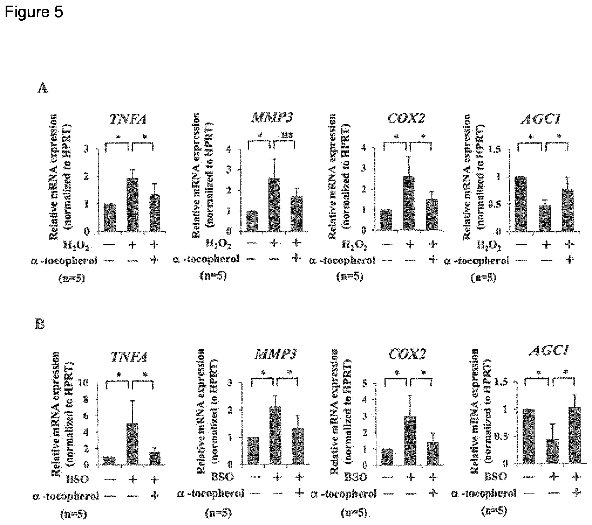

[Figure 5] Figure 5 is a diagram showing the effect of α-tocopherol on oxidative stress. Figure 5A shows that α-tocopherol reduces oxidative stress induced by hydrogen peroxide. Figure 5B shows that α-tocopherol reduces oxidative stress induced by BSO.

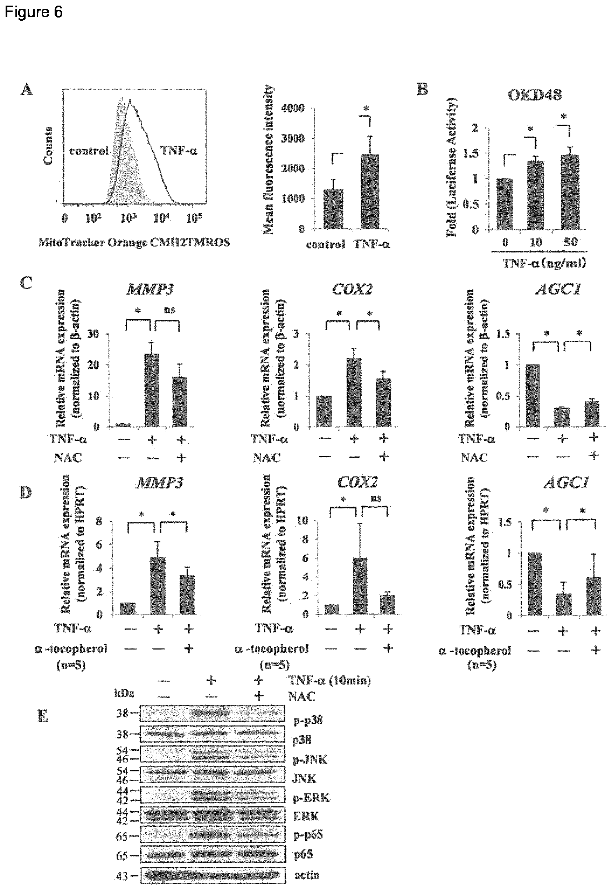

[Figure 6] Figure 6 is a diagram showing response to oxidative stress induced by TNF-**α.** Figures 6A and 6B show that TNF-**α** generates intracellular reactive oxygen species. Figure 6C shows that NAC reduces oxidative stress induced by TNF-**α.** Figure 6D shows that **α-**tocopherol reduces oxidative stress induced by TNF-**α**. Figure 6E shows that NAC suppresses the activation of MAPK by TNF-**α.**

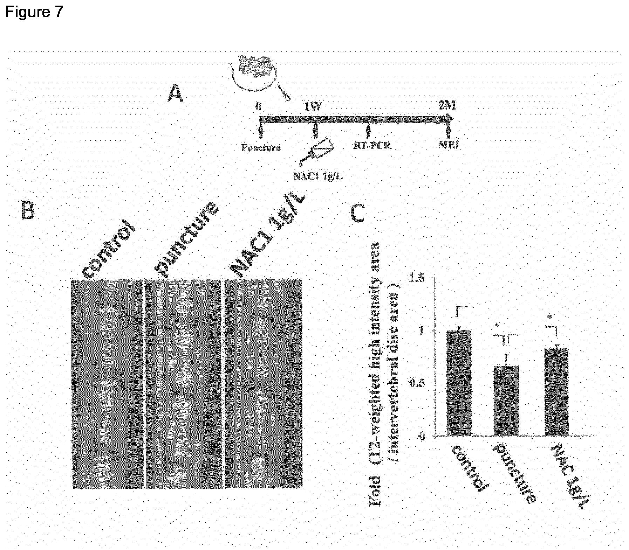

[Figure 7] Figure 7 is a diagram showing the therapeutic effect of NAC on intervertebral disc degeneration in model animals. Figure 7A shows the schedule of the experiment. Figure 7B shows the effect of NAC in MRI. Figure 7C quantitatively shows the MRI results of Figure 7B.

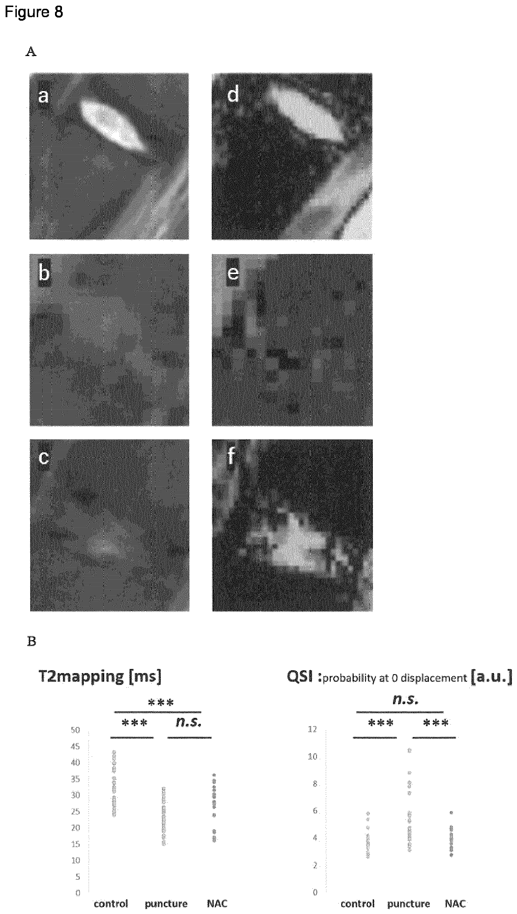

[Figure 8] Figure 8 is a diagram showing that the therapeutic effect of NAC on intervertebral disc degeneration was comparatively studied by a conventional method and QSI. Figure 8A shows a T2-weighted image and a diagram of image analysis by QSI. Figure 8B is a graph showing values obtained by both the analyses.

### Description of Embodiments

In general, pharmaceutical products are divided into those for use in diagnosis, treatment, or prevention according to their purposes. In the present invention, the therapeutic drug refers to a drug that exerts an effect by administration after onset of symptoms, and the prophylactic drug refers to a drug that exerts an effect by administration from before onset of a disease. Typical examples of the therapeutic drug include so-called anticancer drugs. Typical examples of the prophylactic drug include atovaquone/proguanil hydrochloride and mefloquine hydrochloride serving as prophylactic drugs for malaria. Examples of the drug that is used as both therapeutic and prophylactic drugs include Tamiflu which is used in the prevention and treatment of influenza.

In the present invention, NAC for use as an active ingredient can be used directly or after being formulated. Alternatively, a commercially available oral medicine may be used. In the present invention, NAC is administered alone as a therapeutic drug. In a non-claimed aspect NAC may be used in combination with an additional drug. The additional drug that may be used in combination therewith includes an antipain drug such as Loxonin(R), Voltaren(R), Tramal(R), or Tramcet(R). Any drug effective as a therapeutic drug for intervertebral disc degeneration has not yet been developed. There is a need to wait for the further development of a drug that can be expected to have a synergistic or additive effect.

When NAC is formulated alone, the dosage form can be any form of liquid and solid formulations. Usually, a tablet, a capsule or a liquid formation is used. For the tablet, the capsule or the liquid formation, NAC can be administered as an active ingredient at at least 600 mg to 1200 mg/day, i.e., 10 mg/kg/day or more, to an adult. In an experiment using rats as mentioned later, administration at 159 mg/kg/day was effective. Hence, NAC can be administered with this dose as the upper limit. Preferably, 600 mg to 2000 mg can be administered in 3 divided portions per day. It is more preferred to administer 1000 to 1800 mg/day. Also, tocopherol can be administered at 150 mg to 300 mg/day. The dose of NAC for intervertebral disc degeneration can be appropriately selected according to a dosage form, age, body weight, and symptoms on the basis of the doses described above. NAC can usually be formulated by mixing the active ingredient with additives for pharmaceutical use such as a carrier, an excipient, and an auxiliary according to a routine method and formulating the mixture into the desired dosage form.

Tocopherol (not part of the present invention) has 4 isomers of α, β, γ, and δ depending on the positions of methyl groups. Any of these isomers can be used. Also, not only a natural d form but a dl form obtained by synthesis may be used. Among others, α-tocopherol can be suitably used because of being highly active as vitamin E. Tocopherol can also be used as an ester derivative such as tocopherol acetic acid ester, succinic acid ester, or nicotinic acid ester.

Any carrier or excipient may be used. Examples thereof include lactose, corn starch or its derivatives, talc, and steric acid or its salts. Examples of appropriate excipients for use in soft gelatin capsules include plant oils, waxes, fats, semisolid or liquid polyols. Examples of excipients for the preparation of liquid formulations and syrups include water, polyols, saccharose, invert sugar and glucose.

The examination method used in the present invention can not only quantitatively evaluate intervertebral disc degeneration which has been unable to be quantitatively evaluated so far, but can visualize the state thereof. This method also analyzes images of MRI, which is a noninvasive examination method, and therefore reduces burdens on patients. Thus, a therapeutic drug can be administered while its efficacy is studied by monitoring the effect of the therapeutic drug.

The screening method of the present invention can comprise contacting a candidate substance with annulus fibrosus (AF) cells, and using a signal induced or gene or protein expression fluctuating by reactive oxygen species, as an index. Primary annulus fibrosus cells isolated and cultured from a rodent such as a mouse or a rat can be used as the annulus fibrosus cells. Alternatively, human annulus fibrosus cells or the like available as primary cultured cells may be used. Cultured cells that exhibit behavior similar to that of the annulus fibrosus cells in terms of change in gene expression, protein expression, or signaling cascade by oxidative stress can be suitably used.

Any signal induced by reactive oxygen species can be detected. Any of cytokines, such as TNF-**α,** known to be induced by reactive oxygen species, kinases, such as p38, JNK, and ERK, confirmed to be phosphorylated in annulus fibrosus cells due to oxidative stress, nitrotyrosine, and the like as mentioned later may be used as the index. Examples of the gene or protein expression fluctuating by reactive oxygen species include the expression of TNF-**α**, IL-1**β**, MMP-3, COX-2, and aggrecan. A method known in the art, such as quantitative RT-PCR or Northern blotting, which can quantitatively measure RNA can be used for detecting change in gene expression. Particularly, RT-PCR is preferred because this method can sensitively quantify mRNA expression. Also, a method known in the art, such as immunostaining, Western blotting, or ELISA, which can quantify protein expression can be used for quantifying protein expression. In the case of detecting kinases, such as p38, JNK, and ERK, confirmed to be phosphorylated due to oxidative stress, nitrotyrosine, and the like, immunostaining, Western blotting, or ELISA, for example, can be performed using an antibody for phosphorylated kinase detection or an antibody for nitrotyrosine detection. Alternatively, the activation of a kinase of the MAPK signaling cascade, such as p38, JNK, or ERK may be quantified using a substrate known in the art.

A compound found effective by a screening system using cultured cells can be evaluated by the examination method of the present invention using Q-space imaging (hereinafter, also abbreviated to QSI) using intervertebral disc degeneration model animals.

A chemical library generally used may be used as compounds to be screened. Since it is evident that reactive oxygen species is a target, compounds known to reduce oxidative stress induced by reactive oxygen species among existing compounds may be screened to select a compound effective for intervertebral disc degeneration. Further, a compound selected by primary screening using cultured cells may be applied to intervertebral disc degeneration model animals and studied for its effect.

Hereinafter, the present invention will be described in detail with reference to Examples.

### Examples

### [Study using intervertebral disc degeneration model animal]

Model animals prepared by caudal intervertebral disc puncture were studied for whether to possess nature similar to human intervertebral disc degeneration as intervertebral disc degeneration model animals.

### 1. Preparation of intervertebral disc degeneration model animal and results of MRI analysis

The involvement of oxidative stress in intervertebral disc degeneration was studied. The caudal intervertebral discs of 8-week-old Wistar rats were punctured using a 23-G needle to prepare intervertebral disc degeneration models. A group that underwent only development without puncture (sham group) was used as a control group. Two months after the puncture, the T2 intramedullary high-intensity area was measured by MRI (GE Sigma Excite HD 1.5T, manufactured by GE Healthcare Corp.) under the following conditions: TR/TE, 3000/86; echo train length, 12; slice thickness, 2 mm; FOV, 10 cm; matrix size 352X224; NEX, 10 times. The results are shown in Figures 1A and 1B.

The results of T2-weighted MRI showed significantly lower signal intensity in the intervertebral discs of the puncture group than in the intervertebral discs of the control (sham group) (Figure 1A). The ratio of the high-intensity area to intervertebral disc areas of the puncture group was significantly lower than the value of the control group (Figure 1B). The results of MRI indicate that the intervertebral discs of rat caudal intervertebral disc puncture models can serve as intervertebral disc degeneration models for human patients.

Next, annulus fibrosus tissues were harvested, stained with HE (hematoxylin and eosin), and observed (Figure 1C). Smaller nucleus pulposus and less-organized lamellae of the annulus fibrosus were observed in the punctured specimens than in the control.

### 2. RT-PCR analysis

The mRNA expression of the TNF-α (TNFA), MMP-3 (MMP3), COX-2 (COX2), and aggrecan (AGC1), which have been reported to exhibit change by intervertebral disc degeneration was analyzed in intervertebral disc degeneration models.

Total RNA was prepared using RNeasy Mini kit (manufactured by Qiagen N.V.) or TRIzol Reagent (manufactured by Thermo Fisher Scientific Inc.). cDNA was synthesized using Prime Script RT Reagent Kit (manufactured by Takara Bio Inc.).

RT-PCR amplification was performed with Thermal Cycler Dice Real-Time System (manufactured by Takara Bio Inc.) using SYBR Premix Ex Taq (manufactured by Takara Bio Inc.). Expression was normalized to **β-**actin or hypoxanthine phosphoribosyl transferase (HPRT). The results were analyzed by the ddCt method.

The PCR primers used were as follows:
TNF**-α** (F) : 5'-GCAGATGGGCTGTACCTTATC-3' (SEQ ID NO: 1)
   (R) : 5'-GGCTGACTTTCTCCTGGTATG-3' (SEQ ID NO: 2)
MMP-3 (F) : 5'-GGACCAGGGATTAATGGAGATG-3' (SEQ ID NO: 3)
   (R) : 5'-TGAGCAGCAACCAGGAATAG-3' (SEQ ID NO: 4)
COX-2 (F) : 5'-TGAACACGGACTTGCTCACTTTG-3' (SEQ ID NO: 5)
   (R) : 5'-AGGCCTTTGCCACTGCTTGTA-3' (SEQ ID NO: 6)
AGC1 (F) : 5'-GGATCTATCGGTGTGAAGTGATG-3' (SEQ ID NO: 7)
   (R) : 5'-AGTGTGTAGCGTGTGGAAATAG-3' (SEQ ID NO: 8)
**β**-actin (F) : 5'-TGAGAGGGAAATCGTGCGTGAC-3' (SEQ ID NO: 9)
   (R) : 5'-AAGAAGGAAGGCTGGAAAAGAG-3' (SEQ ID NO: 10)
HPRT (F) : 5'-TCCTCATGGACTGATTATGGACA-3' (SEQ ID NO: 11)
   (R) : 5'-TAATCCAGCAGGTCAGCAAAGA-3' (SEQ ID NO: 12)

The results are shown in Figure 1D. The expression of TNF-**α,** MMP-3, and COX-2 whose elevated expression is known to correlates with intervertebral disc degeneration was elevated in the puncture group. Also, the expression of aggrecan whose expression is known to be reduced in intervertebral disc degeneration was found lower in the puncture group than in the control group.

These results of MRI, HE staining, and gene expression analysis by RT-PCR demonstrated that model animals prepared by rat caudal intervertebral disc puncture using a 23-G needle are equivalent to previously reported human intervertebral disc degeneration patients. Thus, it was concluded that these model animals can be used as intervertebral disc degeneration model animals.

### 3. Correlation of oxidative stress with intervertebral disc degeneration in immunohistochemical staining and Western blotting (study using rat model)

The involvement of oxidative stress in intervertebral disc degeneration was analyzed by Western blotting and immunohistochemical staining. The analysis was conducted by detecting oxidative stress markers TNF-**α,** IL-1**β**, and nitrotyrosine.

Immunohistochemical staining was performed by the following method: samples were fixed in 4% paraformaldehyde in phosphate-buffered saline, embedded in paraffin, and then sliced to obtain sections 4 µm thick. TNF-**α** and IL-1**β** were detected after reactions with an anti-TNF-**α** antibody (manufactured by Novus Biologicals) or an anti-IL-1**β** antibody (manufactured by Bioworld Technology, Inc.) as a primary antibody and with HRP-conjugated goat anti-rabbit IgG (manufactured by Sigma-Aldrich Co. LLC) as a secondary antibody. Nitrotyrosine was detected using an anti-nitrotyrosine antibody (manufactured by Abcam plc) as a primary antibody and HRP-conjugated goat anti-mouse IgG (manufactured by Sigma-Aldrich Co. LLC) as a secondary antibody. Detection was performed using diamino benzidine (manufactured by Nacalai Tesque, Inc.). Nuclei were stained with hematoxylin. The results are shown in Figure 1E. Stronger staining of all TNF-**α**, IL-1**β**, and nitrotyrosine was observed in the puncture group than in the control group.

Next, the oxidative stress markers were analyzed by Western blotting. Proteins were extracted using a mammalian protein extraction reagent (manufactured by Pierce/Thermo Fisher Scientific Inc.). The protein extraction buffers and wash buffers used were supplemented with protease inhibitor cocktail (manufactured by F. Hoffmann-La Roche, Ltd.), 1 M NaF, and 50 µM NA₃VO₄. Western blotting was performed by a standard method. Proteins were detected using the antibodies described above and ECL Western Blotting Detection Reagent (manufactured by GE Healthcare Corp.). The control **β**-actin was detected using an anti-**β**-actin antibody (manufactured by Cell Signaling Technology, Inc.).

As shown in Figure 1F, the expression of all TNF-**α,** IL-1**β**, and nitrotyrosine was increased in the puncture group (+) compared with the control group (-), as in the histological staining results. Nitrated tyrosine was quantified by densitometry (lower diagram of Figure 1F). The results showed that more than twice the tyrosine was nitrated in the puncture group compared with the control group.

### 4. Correlation of oxidative stress with intervertebral disc degeneration in immunohistochemical staining (study using human tissue)

Tissues were obtained by surgical operation from 10 patients after obtainment of their informed consent. Eight out of the 10 patients had degenerative lumbar scoliosis, one had degenerative lumbar kyphosis, and one had adult idiopathic scoliosis. A nondegenerative intervertebral disc was obtained from a 17-year-old male sample dissected at autopsy. The degree of intervertebral disc degeneration was determined according to the Pfirrmann's classification.

Immunohistochemical staining was performed in the same way as in the rat models described above. The results are shown in Figure 1G. Higher expression of TNF-**α**, IL-1**β**, and nitrotyrosine was observed in the patient samples of all grades than in healthy samples. Nitrated tyrosine was further quantified by densitometry. The results revealed more than 5 times the frequency of nitrated tyrosine-positives cells in the patient group compared with the healthy individuals (Figure 1H).

### [Response of cultured annulus fibrosus cell to oxidative stress]

Alteration of Gene expression and protein expression that indicated oxidative stress were seen in the rat intervertebral disc degeneration models and the human intervertebral disc degeneration patients. Hence, alteration brought about by oxidative stress was analyzed using cultured cells. First, gene expression known to fluctuate in intervertebral disc degeneration was analyzed using rat annulus fibrosus cells applied oxidative stress.

### 1. Preparation of rat annulus fibrosus cell

Rat annulus fibrosus cells were prepared as follows: rat annulus fibrosus tissues were dissected from the lumbar and coccyx intervertebral discs of 8-week-old female Wistar rats, treated with DMEM containing 0.04% pronase E (manufactured by SERVA Electrophoresis GmbH) at 37°C for 1 hour, and subsequently treated with 0.025% collagenase P (manufactured by Roche Diagnostics K.K.) at 37°C for 4 hours for isolation. The isolated cells were washed with Dulbecco's modified Eagle's medium (DMEM, manufactured by Invitrogen Corp.) containing 5% heat-inactivated fetal bovine serum (manufactured by JRH Biosciences Lenexa). The isolated annulus fibrosus cells were cultured in DMEM containing 10% fetal bovine serum and 1% penicillin-streptomycin under conditions of 5% CO₂ at 37°C. The cells were used within the first five passages for analysis.

### 2. Increase in intracellular level of reactive oxygen species by oxidative stress

Annulus fibrosus cells were treated with an oxidative stress inducer hydrogen peroxide (manufactured by Wako Pure Chemical Industries, Ltd.) or a glutathione synthesis inhibitor buthionine sulfoximine (BSO, manufactured by Sigma-Aldrich Co. LLC), and the intracellular level of reactive oxygen species (ROS) was measured.

The cells were treated with 100 µM hydrogen peroxide or 1 mM BSO for 24 hours. Reactive oxygen species were stained with MitoTracker Orange CMH2TM ROS (manufactured by Life Technologies Corp.) for 30 minutes and measured using a cell sorter (FACSCalibur, manufactured by Becton-Dickinson Immunocytometry Systems) (Figure 2A). Both the hydrogen peroxide treatment and the BSO treatment were shown to increase the intracellular level of reactive oxygen species.

### 3. Alteration in gene expression by oxidative stress

TNF-**α**, MMP-3, COX-2, and aggrecan which exhibited alteration in gene expression in the intervertebral disc degeneration model animals were analyzed for gene expression under oxidative stress induced by hydrogen peroxide or BSO.

Rat annulus fibrosus cells were cultured in a medium added with hydrogen peroxide (concentration: 10 µM and 100 µM) or BSO (concentration: 0.2 mM and 1 mM) for 24 hours and collected. RNA was extracted, and the mRNA expression of TNF-**α**, MMP-3, COX-2, and aggrecan was quantitatively measured by RT-PCR. RNA extraction and RT-PCR were performed in the same way as in the model animals described above. Figure 2B shows the results obtained by the addition of hydrogen peroxide, and Figure 2C shows the results obtained by the addition of BSO.

A concentration-dependent increase in the mRNA expression of TNF-**α**, MMP-3, and COX-2 was observed both in the medium added with hydrogen peroxide and in the medium added with BSO. Also, the mRNA expression of aggrecan was reduced in a concentration-dependent manner. Alteration of gene expression similar to that in the rat intervertebral disc degeneration models was also shown using cultured cells.

### [Study on oxidative stress signal]

### 1. Involvement of MAPK

Downstream signals of oxidative stress were studied. The involvement of MAPKs (p38, ERK, and JNK) in the downstream system of signals induced by reactive oxygen species was analyzed. Annulus fibrosus cells were treated with 100 µM hydrogen peroxide, collected 0, 5, 10, 30, or 60 minutes later, and analyzed by Western blotting. Protein extraction and Western blotting were performed in the same way as in the experiment using the animal models described above.

Detection was performed using an anti-p38 antibody, an anti-phosphorylated p38 antibody, an anti-JNK antibody, an anti-phosphorylated JNK antibody, an anti-ERK antibody, an anti-phosphorylated ERK antibody, an anti-NF-KB p65 antibody, an anti-phosphorylated NF-κB p65 antibody, or an anti-**β**-actin antibody (all manufactured by Cell Signaling Technology, Inc.) as a primary antibody, a HRP-conjugated goat anti-rabbit antibody (manufactured by Sigma-Aldrich Co. LLC) as a secondary antibody, and ECL Western Blotting Detection Reagent. All the MAPKs p38, ERK, and JNK were strongly phosphorylated 10 minutes after hydrogen peroxide treatment (Figure 3A).

Likewise, downstream signals of oxidative stress were studied by the addition of BSO. Annulus fibrosus cells were treated with 1 mM BSO, collected 0, 5, 10, 30, or 60 minutes later, and analyzed by Western blotting. All the MAPKs p38, ERK, and JNK were strongly phosphorylated 10 minutes after BSO treatment, as in the hydrogen peroxide treatment (Figure 3B).

### 2. Effect of MAPK inhibitor on COX-2 gene expression

The obtained results show that oxidative stress was transduced into cells via the MAPK signaling cascade. Hence, MAPK inhibitors were analyzed for whether to influence gene expression under oxidative stress.

Hydrogen peroxide or BSO was added to rat annulus fibrosus cells in the presence of MAPK inhibitors, and their effects on an increase in COX-2 mRNA expression were analyzed. The MAPK inhibitors used were PD98059 (ERK inhibitor; represented by PD in Figure 3), SP600125 (JNK inhibitor; represented by SP in Figure 3), and SB203580 (p38 inhibitor; represented by SB in Figure 3) (all manufactured by Wako Pure Chemical Industries, Ltd.). These MAPK inhibitors were each added at concentration of 10 µM to a medium, and 30 minutes later, 100 µM hydrogen peroxide or 1 mM BSO was added thereto. The expression of COX-2 was analyzed by RT-PCR 24 hours later.

The expression of COX-2 was significantly suppressed by the addition of any MAPK inhibitor of the ERK inhibitor (PD), the JNK inhibitor (SP), and the p38 inhibitor (SB) (Figures 3C and 3D). From these results, MAPK was confirmed to be responsible for the downstream signaling mechanism of oxidative stress.

### [Effect of antioxidant]

### 1. Effect of NAC on oxidative stress

Oxidative stress causes gene expression seen in intervertebral disc degeneration. Hence, the effect of an antioxidant was examined. An antioxidant NAC along with oxidative stress was added and analyzed for how the antioxidant would influence gene expressions that were changed in intervertebral disc degeneration.

100 µM hydrogen peroxide or 1 mM BSO was added to rat annulus fibrosus cells in the presence of 100 µM NAC, and the cells were cultured for 24 hours, followed by the measurement of the mRNA expression of TNF-**α**, MMP-3, COX-2, and aggrecan (Figures 4A and 4B).

NAC added to a medium significantly suppressed an increase in the expression of TNF-**α**, MMP-3, and COX-2 by oxidative stress induced by any of the drugs hydrogen peroxide and BSO. Also, NAC suppressed reduction in the expression of aggrecan by oxidative stress. These results indicate that NAC reduces oxidative stress in cultured cells and suppresses change in genes specifically expressed in intervertebral disc degeneration.

### 2. Effect of NAC on MAPK signaling cascade against oxidative stress

Hydrogen peroxide was added to rat annulus fibrosus cells, and the kinase phosphorylation of the MAPK signaling cascade was analyzed 15 minutes later (Figures 4C and 4D). The cells were treated with 100 µM hydrogen peroxide in the presence of 100 µM NAC, collected cells 15 minutes later, and analyzed by Western blotting. NAC addition significantly suppressed the phosphorylation of p38 MAPK. This suggests that the antioxidant NAC is particularly effective for the p38 MAPK signaling cascade and influences gene expression via this signaling cascade.

### 3. Effect of tocopherol on gene expression against oxidative stress

The antioxidant NAC was found effective for gene expression and the signaling cascade. Hence, other antioxidants were also analyzed. Tocopherol, a well-known antioxidant, was used in the analysis.

Hydrogen peroxide or BSO was added to rat annulus fibrosus cells in the presence of 20 mM **α**-tocopherol, and the cells were cultured for 24 hours, followed by the measurement of the mRNA expression of TNF-**α**, MMP-3, COX-2, and aggrecan (Figures 5A and 5B). Here, ethanol was used as a solvent for **α**-tocopherol. Cells cultured in a medium added only with ethanol were used as a control.

**α**-tocopherol added to a medium significantly suppressed an increase in the expression of TNF-**α**, MMP-3, and COX-2 by oxidative stress induced by any of the drugs hydrogen peroxide and BSO. Also, **α**-tocopherol suppressed reduction in the expression of aggrecan by oxidative stress. Not only NAC but **α**-tocopherol was found effective for alteration of gene expression observed in intervertebral disc degeneration, suggesting that antioxidants have a therapeutic effect on intervertebral disc degeneration.

### [Suppression of intracellular reactive oxygen species by antioxidant]

Antioxidants were analyzed for whether to suppress intracellular reactive oxygen species. An inflammatory cytokine TNF-**α** (manufactured by R&D Systems, Inc.) was added at a concentration of 50 ng/mL to a medium of rat annulus fibrosus cells, and an increase in the intracellular level of reactive oxygen species was quantified using MitoTracker in the same way as above. TNF-**α** addition significantly increased the intracellular level of reactive oxygen species.

Next, oxidative stress was quantified using a plasmid OKD48 (P(3xARE)TKbasal-hNrf2(1-433)-GL4-F) (Non Patent Literature 6). OKD48 is a plasmid constructed so as to specifically respond to oxidative stress and can be used in the monitoring of *in vivo* oxidative stress. Rat annulus fibrosus cells were transfected with 300 ng of OKD48 and 200 ng of pRL-TK using LipofectAMINE 3000 (manufactured by Invitrogen Corp.). On the next day, the cells were treated with 10 ng/ml or 50 ng/ml of TNF-**α** for 10 minutes and collected, followed by the quantification of activity using Dual-Luciferase Reporter Assay System (manufactured by Promega Corp.).

As shown in Figure 6B, TNF-**α** increased luciferase activity in a concentration-dependent manner, suggesting that TNF-**α** generates oxidative stress. An increase in the level of reactive oxygen species in rat annulus fibrosus cells by the physiological stimulus of TNF-**α** indicates that the rat annulus fibrosus cells well reflect physiological phenomena of intervertebral disc degeneration.

Next, NAC or tocopherol was analyzed for whether to reduce oxidative stress induced by TNF-**α** by its addition. 50 ng/ml of TNF-**α** was added to cells in the presence of 100 µM NAC or 20 mM **α**-tocopherol, and the cells were collected 24 hours later, followed by gene expression analysis on MMP-3, COX-2, and aggrecan by RT-PCR (Figures 6C and 6D). NAC and tocopherol were also shown to suppress oxidative stress induced by the addition of TNF-**α**.

NAC was also analyzed for whether to suppress phosphorylation in the MAPK signaling cascade generating downstream signals of oxidative stress (Figure 6E). In the same way as above, cells were treated with 50 ng/ml TNF-**α** in the presence of 100 µM NAC and analyzed by Western blotting. NAC also suppressed the activation of the MAPK signaling cascade caused by stimulation with TNF-**α**.

### [Therapeutic effect of NAC on intervertebral disc degeneration in animal model]

These results obtained using animal models of intervertebral disc degeneration, the analysis of patients' tissues, and cultured cells revealed that oxidative stress is involved in intervertebral disc degeneration. Also, the analysis using cultured cells revealed that the antioxidants NAC and tocopherol suppress gene expression, protein expression, and a signaling cascade that correlate with intervertebral disc degeneration. Accordingly, whether antioxidants are effective for the treatment of intervertebral disc degeneration was analyzed using animal models.

The caudal intervertebral discs of 8-week-old female Wistar rats were punctured using a 23-G needle to prepare intervertebral disc degeneration models. The animals were divided into a group given NAC water (1 g/L) from 1 week after the puncture (NAC group) and a group raised with normal water (puncture group). A group that underwent only development without puncture (control group) was used as a control. Whether or not the administration of NAC would suppress intervertebral disc degeneration was studied 2 months after the puncture (see Figure 7A). The average body weight of the rats during the administration of NAC water was 220 g, and the average water intake of 24 hours was 35 g; thus, the rats took 159 mg/kg/day of NAC on average per day. The items to be studied were histological evaluation and a T2 intramedullary high-intensity area by MRI. As a result of analyzing MRI images obtained 2 months after the puncture, the T2 high-intensity area was significantly improved in the NAC group compared with the puncture group. NAC administered 1 week after treatment of inducing intervertebral disc degeneration, i.e., after development of intervertebral disc degeneration, was effective, indicating that the administration of NAC can serve as a method for treating intervertebral disc degeneration.

Tocopherol, which was able to produce similar effects in the experiment using annulus fibrosus cells, can also be expected to have a therapeutic effect similar to that of NAC. In addition, any drug suppressing oxidative stress via the MAPK signaling cascade is thought to have the mechanism of action similar to that of NAC or tocopherol and can therefore be expected to have a therapeutic effect on intervertebral disc degeneration.

### [Examination method using QSI]

A novel examination method using QSI will be described. The degrees of intervertebral disc degeneration are classified to grades 1 to 5 according to the Pfirrmann's classification to determine the severity, under the present circumstance. The Pfirrmann's classification is a qualitative evaluation method and is not suitable for grasping slight change. The evaluation of therapeutic effects on intervertebral disc degeneration requires a quantitative evaluation method using images.

QSI is a MRI analysis method originally reported as a method for analyzing porous substances (Non Patent Literature 7). Diffusion weighted imaging is currently used in clinical practice. QSI is an imaging method of obtaining data by varying the b value setting of the diffusion weighted imaging. Its application to biological information has been started in recent years because QSI analysis permits obtainment of microsized structure information (Non Patent Literatures 8 and 9).

The analysis of the spinal cord by use of QSI has also been attempted, and it has been reported that white matter disorder or daily change in intervertebral disc can be detected (Non Patent Literatures 10 and 11). It has also been reported as to intervertebral disc degeneration that the application of QSI correlates with classification based on the qualitative evaluation method Pfirrmann's grading (Non Patent Literature 12). The evaluation of Non Patent Literature 12 was conducted using a QSI parameter kurtosis. By contrast, the present inventors further optimized an analysis method and an imaging protocol and enabled more minute change to be grasped by using probability at zero displacement or fractional anisotropy of kurtosis as a parameter. As a result, even slight improvement in disease by treatment can be detected.

The present inventors prepared rat intervertebral disc degeneration models in the same way as above and conducted QSI analysis. Here, the QSI analysis was conducted using our uniquely developed program, though the analysis can employ software such as dTV II FZR. A puncture group, a NAC group, and a control group each involving 5 rats were subjected to the respective procedures and analyzed. Figure 8A shows the comparison between the conventional method T2-weighted MRI (Figure 8A, a to c) and QSI: probability at zero displacement (Figure 8A, d to f). The comparison of analysis results among the control group (a and d), the puncture group (b and e), and the NAC group (c and f) is shown.

The T2-weighted images showed the difference between the control group (a) and the puncture group (b) or the NAC group (c), but rarely showed the difference between the puncture group (b) and the NAC group (c). However, the QSI analysis showed the difference between the puncture group (e) and the NAC group (f). Thus, findings on the prevention of degeneration by NAC can be grasped by QSI more sensitively.

Figure 8B shows a graph of summarizing T2 mapping values obtained by analyzing T2-weighted images focused on the nucleus pulposus in intervertebral discs, and QSI parameter values. The values obtained from the T2-weighted images did not significantly differ between the puncture group and the NAC group, whereas the QSI parameter values obtained by the QSI analysis exhibited significant difference between the puncture group and the NAC group, indicating the therapeutic effect of NAC. Here, the results obtained using model animals are shown. However, results obtained using intervertebral disc degeneration patients also show that change can be grasped more sensitively. Thus, the degree of degeneration and changes in symptoms can be quantitatively analyzed sensitively.

The examination method of the present invention has the advantages that this method can not only detect even slight difference sensitively but can monitor the course of a disease as quantitative numerical values. Furthermore, the degree of the disease dose not differ among physicians or among medical institutes because the degree of the disease can be evaluated as quantitative numerical values.

### Industrial Applicability

Antioxidants NAC and tocopherol can be used in the treatment of intervertebral disc degeneration. The antioxidants are also considered effective as therapeutic drugs for diseases, such as low back pain and disc herniation, caused by intervertebral disc degeneration as a factor. Moreover, since the present inventors have revealed that oxidative stress is responsible for intervertebral disc degeneration, a novel screening method can be provided. The examination method disclosed herein permits accurate diagnosis because this method can not only quantify minute change that can be grasped by MRI but can be insusceptible to the difference in the backgrounds of MRI images.

## Claims

1. A therapeutic drug for use in a method of treating intervertebral disc degeneration and/or a drug for use in a method of improving low back pain by oral administration comprising an antioxidant as the only active ingredient, and
the antioxidant is N-acetylcysteine, and
is administered at a dose of 10 mg/kg/day to 159 mg/kg/day.

2. A method for examining a therapeutic effect of the therapeutic drug for intervertebral disc degeneration and/or the low back pain improving drug according to claim 1, comprising analyzing MRI images obtained before and after treatment by Q-space imaging using Probability at zero displacement or Fractional Anisotropy of Kurtosis as a parameter and comparatively analyzing the quantified values.

3. A method for screening for a therapeutic drug for intervertebral disc degeneration, and/or a low back pain improving drug, comprising:
contacting a candidate substance with annulus fibrosus (AF) cells under induced oxidative stress;
using a signal induced by reactive oxygen species as an index, and
further allowing a rodent intervertebral disc degeneration model animal that has developed intervertebral disc degeneration, to take the candidate substance selected by the screening method; and
making a selection by using a signal induced by reactive oxygen species as an index.

4. The method for screening for a therapeutic drug for intervertebral disc degeneration and/or a low back pain improving drug according to claim 3, further comprising:
obtaining MRI images of the rodent intervertebral disc degeneration model animal before and after the administration of the candidate substance,
analyzing the obtained MRI images by Q-space imaging using Probability at zero displacement or Fractional Anisotropy of Kurtosis as a parameter; and
comparatively analyzing the quantified values to determine the efficacy of the candidate substance.

## Patentansprüche

1. Therapeutischer Wirkstoff zur Verwendung in einem Verfahren zur Behandlung von Bandscheibendegeneration und/oder ein Wirkstoff zur Verwendung in einem Verfahren zur Verbesserung von Schmerzen im unteren Rückenbereich durch orale Verabreichung, umfassend ein Antioxidans als den einzigen Wirkstoff, und
das Antioxidans N-Acetylcystein ist, und
in einer Dosis von 10 mg/kg/Tag bis 159 mg/kg/Tag verabreicht wird.

2. Verfahren zur Untersuchung einer therapeutischen Wirkung des therapeutischen Wirkstoffs für Bandscheibendegeneration und/oder des den Schmerz im unteren Rückenbereich verbessernden Wirkstoffs nach Anspruch 1, umfassend das Analysieren von MRT-Bildern, die vor und nach der Behandlung durch Q-Raum-Bildgebung erhalten wurden, unter Verwendung der Wahrscheinlichkeit bei Nullpunktverschiebung oder der fraktionellen Anisotropie der Kurtosis (engl.: "Fractional Anisotropy of Kurtosis") als Parameter und vergleichendes Analysieren der quantifizierten Werte.

3. Verfahren zum Screening nach einem therapeutischen Wirkstoff für Bandscheibendegeneration und/oder einem Wirkstoff zur Verbesserung von Schmerzen im unteren Rückenbereich, umfassend
Inkontaktbringen einer Kandidatensubstanz mit Anulus fibrosus (AF)-Zellen unter induziertem oxidativem Stress;
Verwenden eines durch reaktive Sauerstoffspezies induzierten Signals als Index, und
ferner Ermöglichen, dass ein Nagetier-Bandscheibendegenerations-Tiermodell, das eine Bandscheibendegeneration entwickelt hat, die durch das Screening-Verfahren ausgewählte Kandidatensubstanz aufnimmt; und
Treffen einer Auswahl unter Verwendung eines durch reaktive Sauerstoffspezies induzierten Signals als Index.

4. Verfahren zum Screening nach einem therapeutischen Wirkstoff für Bandscheibendegeneration und/oder einem Wirkstoff zur Verbesserung von Schmerzen im unteren Rückenbereich nach Anspruch 3, ferner umfassend
Erhalten von MRT-Bildern des Nagetier-Bandscheibendegenerations-Tiermodells vor und nach der Verabreichung der Kandidatensubstanz,
Analysieren der erhaltenen MRT-Bilder durch Q-Raum-Bildgebung unter Verwendung der Wahrscheinlichkeit bei Nullpunktverschiebung oder der fraktionellen Anisotropie der Kurtosis als Parameter; und
vergleichendes Analysieren der quantifizierten Werte, um die Wirksamkeit der Kandidatensubstanz zu bestimmen.

## Revendications

1. Médicament thérapeutique destiné à être utilisé dans un procédé de traitement de la dégénérescence des disques intervertébraux et/ou médicament destiné à être utilisé dans un procédé d'amélioration des douleurs lombaires par une administration par voie orale comprenant un antioxydant en tant que seul principe actif, et
l'antioxydant est le N-acétylcystéine, et
est administré à une dose allant de 10 mg/kg/jour à 159 mg/kg/jour.

2. Procédé d'évaluation d'un effet thérapeutique du médicament thérapeutique destiné au traitement de la dégénérescence des disques intervertébraux et/ou du médicament destiné à améliorer les douleurs lombaires selon la revendication 1, comprenant l'analyse d'images obtenues par IRM avant et après le traitement par imagerie de l'espace Q en utilisant la probabilité à un déplacement nul ou l'anisotropie fractionnelle de Kurtosis comme paramètre et en analysant comparativement les valeurs quantifiées.

3. Procédé de criblage destiné à un médicament thérapeutique pour le traitement de la dégénérescence des disques intervertébraux, et/ou un médicament permettant d'améliorer les douleurs lombaires, comprenant :
la mise en contact d'une substance candidate avec des cellules de l'anneau fibreux (AF) sous stress oxydatif induit ;
l'utilisation d'un signal induit par des espèces réactives de l'oxygène en tant qu'indice, et
le fait de permettre en outre à un modèle animal rongeur à dégénérescence des disques intervertébraux qui a développé une dégénérescence des disques intervertébraux de prendre la substance candidate choisie par le procédé de criblage ; et
la réalisation d'une sélection en utilisant un signal induit par des espèces réactives de l'oxygène en tant qu'indice.

4. Procédé de criblage destiné à un médicament thérapeutique pour le traitement de la dégénérescence des disques intervertébraux et/ou un médicament destiné à améliorer les douleurs lombaires selon la revendication 3, comprenant en outre :
l'obtention d'images par IRM du modèle animal rongeur à dégénérescence des disques intervertébraux avant et après l'administration de la substance candidate,
l'analyse des images obtenues par IRM par l'imagerie de l'espace Q en utilisant la probabilité à un déplacement nul ou l'anisotropie fractionnelle de Kurtosis comme paramètre ; et
l'analyse comparative des valeurs quantifiées pour déterminer l'efficacité de la substance candidate.


### SEQUENCE LISTING

<110> Keio University
<120> Therapeutic agent for intervertebral disc degeneration
<130> S15046PCT
<160> 12
<170> PatentIn version 3.5
<210> 1
   <211> 21
   <212> DNA
   <213> Artificial Sequence
<220>
   <223> PCR primer
<400> 1
   gcagatgggc tgtaccttat c 21
<210> 2
   <211> 21
   <212> DNA
   <213> Artificial Sequence
<220>
   <223> PCR primer
<400> 2
   ggctgacttt ctcctggtat g 21
<210> 3
   <211> 22
   <212> DNA
   <213> Artificial Sequence
<220>
   <223> PCR primer
<400> 3
   ggaccaggga ttaatggaga tg 22
<210> 4
   <211> 20
   <212> DNA
   <213> Artificial Sequence
<220>
   <223> PCR primer
<400> 4
   tgagcagcaa ccaggaatag 20
<210> 5
   <211> 23
   <212> DNA
   <213> Artificial Sequence
<220>
   <223> PCR primer
<400> 5
   tgaacacgga cttgctcact ttg 23
<210> 6
   <211> 21
   <212> DNA
   <213> Artificial Sequence
<220>
   <223> PCR primer
<400> 6
   aggcctttgc cactgcttgt a 21
<210> 7
   <211> 23
   <212> DNA
   <213> Artificial Sequence
<220>
   <223> PCR primer
<400> 7
   ggatctatcg gtgtgaagtg atg 23
<210> 8
   <211> 22
   <212> DNA
   <213> Artificial Sequence
<220>
   <223> PCR primer
<400> 8
   agtgtgtagc gtgtggaaat ag 22
<210> 9
   <211> 22
   <212> DNA
   <213> Artificial Sequence
<220>
   <223> PCR primer
<400> 9
   tgagagggaa atcgtgcgtg ac 22
<210> 10
   <211> 22
   <212> DNA
   <213> Artificial Sequence
<220>
   <223> PCR primer
<400> 10
   aagaaggaag gctggaaaag ag 22
<210> 11
   <211> 23
   <212> DNA
   <213> Artificial Sequence
<220>
   <223> PCR primer
<400> 11
   tcctcatgga ctgattatgg aca 23
<210> 12
   <211> 22
   <212> DNA
   <213> Artificial Sequence
<220>
   <223> PCR preimer
<400> 12
   taatccagca ggtcagcaaa ga 22